# EUROPEAN PATENT APPLICATION

(11) **EP 1 142 547 A1**
(43) Date of publication of application: **10.10.2001**
(21) Application number: 01107806.0
(22) Date of filing: 05.04.2001
(51) Int. Cl.: A61F 13/64, A61F 13/15

(54) **Absorbent articles for various torso sizes**

(30) Priority: 06.04.2000 US 544092
(71) Applicant: First Quality Enterprises, Inc., McElhattan, Pennsylvania 17748 (US)
(72) Inventor: Damaghi, Kambiz, Kings Point, New York 11021 (US); Hamzeh, Karami, McElhattan, PA 17748 (US)
(74) Representative: Motsch, Andreas

(57) **Abstract**

A disposable absorbent article (10) is provided which is adapted to fit various torso sizes. The article comprises an elasticated belt (11) and an insert (31), which may be formed integrally with the belt (11), or may constitute a separate member. The belt (11) and the insert (31) are provided with complementary hooks (41,43) and loops (27,29), and are sized so that, in use, the absorbent article (10) fits tightly to different torso sizes.

## Description

### RELATED APPLICATIONS

This application is a continuation-in-part of application serial number 09/376,282 filed August 18, 1999, which is, in turn, a continuation-in-part of application serial number 09/149,265 filed September 8, 1998, which is, in turn, a continuation-in-part of application serial number 09/097,198 filed June 12, 1998.

### FIELD OF THE INVENTION

The present invention relates generally to absorbent articles such as disposable diapers, infant training pants, adult undergarments and incontinence briefs used for absorption and containment of urine and other body exudates. In one aspect, the present invention relates to such absorbent articles which assure improved body fit and prevent leakage of fluids and body exudates during their use. In a more particular aspect, the absorbent articles of the present invention are designed to fit comfortably about different size torsos, easy to wear, change and remove, less bulky than the prior art type absorbent articles, and which can be more readily mass produced economically.

### BACKGROUND OF THE INVENTION

Ever since infant diapers and adult incontinence briefs began to find widespread institutional and household use, more and more attention has been focused on improving body fit and comfort, leakage prevention, ease of wear and removal, and simplicity and economy of their mass production. In case of adult incontinent persons, varying hip, waist and torso sizes often dictates the relative dimensions and different constructions of the brief with a view to assuring fitness, comfort and leak prevention. In addition, the simplicity of such varied size briefs plays a major practical role from the standpoint of economy of their production and utilization.

Current disposable briefs are frequently oversized in order to fit persons with pre-determined hip and waist girth sizes. For example, a medium size brief is usually designed for persons having a waist-hip size of 32-44 inches. These briefs are provided with adhesive tabs to secure the brief on the body of the wearer. Since these briefs are intended to be worn by persons having different girth sizes, they are provided with adjustable adhesive tape tabs in order to assure satisfactory and comfortable fit. For persons with larger girth sizes, more material is required to form the brief than is necessary, thus contributing to waste in economy of fabrication. The problem is even more acute when forming extra large briefs for persons with girth sizes of 56-64 inches.

Although most present day briefs contain copious amounts of superabsorbent polymer (SAP) and fluff material, the user still experiences fluid leak and discomfort. The introduction of so-called "pull-up", also known as "pull-on" type briefs in recent years was designed to alleviate some of the problems, however, these briefs are often difficult to put on an incontinent person. Even more difficulty is experienced when removing pull-up briefs when they have been soiled. In order to assure body tight fitness, the prior art briefs employ hook and loop fastening systems comprising hooks and loops wherein the hooks are defined by tape tab-like structures and the loops are defined by special substrates defining landing zones onto which the hooks can be securely engaged. Ordinarily, a plurality of complementary hooks and loops are needed to afford a tight fit around the torso. The material used for the loop component of the fastening system, i.e., the loop type landing zones, are so expensive that makes it cost prohibitive to manufacture baby diapers, and even more cost prohibitive for making adult briefs since they require the use of more landing zone materials.

In the aforementioned commonly assigned application serial number 09/376,282 there is described a generally T-shaped brief and L-shaped brief designed to provide greater degree of fluid leak prevention while reducing the material required to fabricate the brief. The brief described therein is generally one-piece integral disposable article having a cross-piece and an elongated member which, in a stretched view, displays a generally T-shaped, or L-shaped configuration. The briefs described in this application constitute improvement over the prior art briefs, including those described in the aforementioned application.

Therefore, it is an object of this invention to provide baby diapers and adult incontinent briefs with improved hook and loop fastening systems which assures tight body fit for various torso sizes.

It is another object of this invention to provide such diapers, briefs and other absorbent garments for use by incontinent persons which are provided with unique hook and loop fastening systems designed to assure maximum comfort and prevention of leakage of body fluids and exudates.

It is a further object of this invention to provide diapers, briefs and absorbent articles of the types herein described having uniquely designed and arranged complementary hook and loop fastening systems which minimize the need to use expensive loop type landing zone materials, and hence afford more economical means of manufacturing these absorbent articles.

It is still an object of this invention to provide such absorbent articles which can be readily put on by an incontinent person with minimal discomfort and inconvenience, and which can be removed when soiled without the problems normally experienced in case of putting on or removal of soiled briefs of the prior art variety.

The foregoing and other objects and advantageous features of the absorbent articles of the present invention will be more fully appreciated from the following detailed description and the accompanying drawings.

### SUMMARY OF THE INVENTION

The disposable absorbent article of the present invention can be formed using minimum quantity of expansive material, comprising an elasticated belt which, in use, is adapted to fits snugly around and to the different individual torso sizes. The belt is used in combination with an insert member, which together may form an integral article, or they may be separate pieces adapted to be used together in the same manner as the integral article. The insert is formed of various layers as in the prior art absorbent articles. The belt and the insert are provided with complementary hooks and loops strategically positioned so that when the article is worn, the hooks and loops engage in one another to firmly and comfortably secure the article around the waist of the user.

### BRIEF DESCRIPTION OF THE DRAWINGS

In the drawings, wherein like reference numerals are employed to designate like parts:
Figure 1 is a plan view of the diaper of this invention in a fully stretched position;
Figure 2 is a perspective view of the diaper illustrated in Figure 1;
Figure 3 is a perspective view similar to Figure 2 showing complementary hooks and loops in inter-engaged positions when the article is worn by the user, and
Figure 4 is a stretched plan view of the belt component of the diaper of this invention wherein a portion of the belt is elasticated by a plurality of spaced apart, generally parallel elastic elements, and
Fig. 5 is a sectional view taken along the lines 5-5 of Figure 4.

### DETAILED DESCRIPTION OF THE PREFERRED EMBODIMENT OF THE INVENTION

In the ensuing detailed description, the present invention will be described by reference to a diaper and specifically illustrated as a generally T-shaped diaper. However, it must be understood that this description is applicable to like absorbent articles such as incontinence briefs, training pants and the like articles of the same or different configuration.

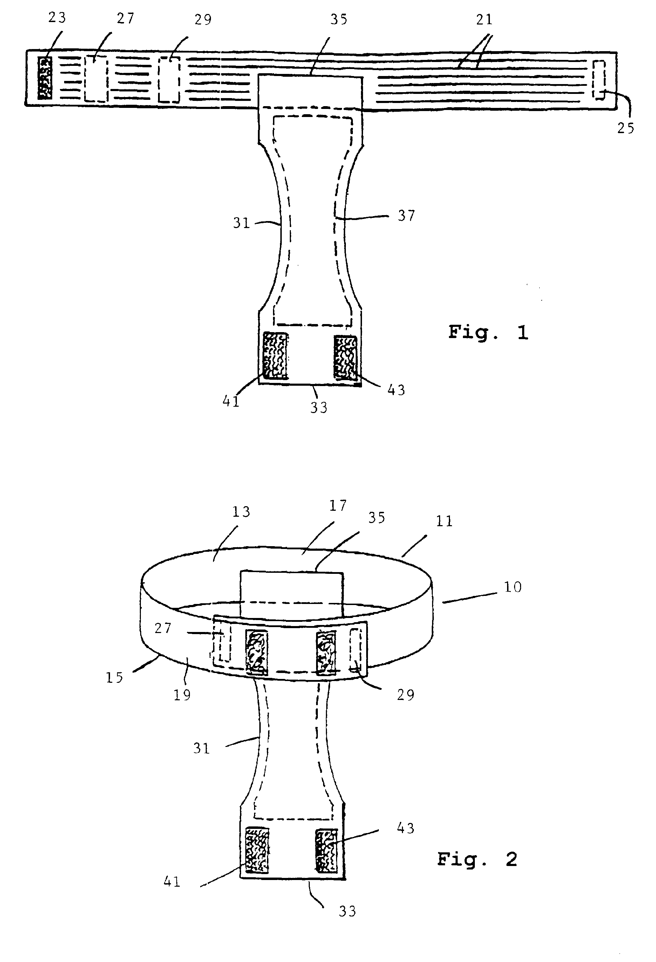

Thus, referring to Figures 1-3, the diaper of this invention is designated by 10 and comprises an elasticated belt member 11 adapted to be wrapped around the waist of the wearer. The belt 11 has a back waist portion 13 and a front waist portion 15, each comprising a bodyside surface 17 and an opposed surface 19 away from the body side of the wearer. The belt 11 is made of spunbond nonwoven stretchable material. Thus, the belt 11 is made stretchable by a plurality of spaced apart, generally parallel elastic elements or strings 21 (see Figure 4) running the cross (horizontal) direction. Alternatively, the belt 11 may be made from an elastomeric material which itself is inherently elastic, in which case no elastic elements are needed for imparting elasticity to the belt. Such elastomeric materials are well known in the art, including natural and synthetic rubbers. In its preferred embodiment, the belt 11 exhibits elasticity which varies from about 50 to about 500, preferably from about 100 to about 300 percent. When a belt of such degree of elasticity is used, less of the expensive nonwoven material will be required to form the diaper, with consequent reduction in cost and economy of manufacture. In addition, one size product can be made to fit persons of different hip/waist torso sizes.

The bodyside surface 17 of the belt 11 is provided with a hook 23 formed of a Velcro strip attached at one end of the belt, and a loop 25 is disposed at the other end of the body side surface 19, as shown in Figures 1 and 4. A pair of spaced apart, generally parallel disposed loops 27, 29 are located on the opposed surface 19 of the belt and are adapted to provide female receiving zones when the belt 11 is wrapped around the waist.

The diaper 10 also comprises an insert 31, having a free distal end 33 away from the belt 11 when the diaper is viewed in the stretched flat position, and a proximal end 35 which is attached to the belt 11 using conventional adhesives, heat, ultrasonic energy or other known conventional methods. The insert 31 is similar to conventional disposable briefs and comprises a coversheet, a backsheet and an absorbent core disposed between the coversheet and the backsheet. The materials or nonwoven films used in making the diapers of this invention are, generally, of type and variety known in the prior art as, for example, described in the aforementioned copending application serial number 09/149,265, the disclosure of which is fully incorporated herein by reference. Thus, the coversheet may be made of a liquid pervious, soft compliant material which is non-irritating and is skin friendly. By way of examples, such materials include porous foams, reticulated foams, plastics, natural fibers such as woods or cotton fibers, synthetic fibers made of polyester or poly-propylene available from First Quality Fibers, Inc., McElhattan, PA, or made from a suitable combination of said materials.

The absorbent core may be formed from a wide variety of liquid absorbent materials of the type used in making absorbent disposable diapers and other absorbent articles. This core may be made of wood pulp fibers and superabsorbent polymers (SAP) such as IM 7000 series available from Clarian Products, Inc., Portsmouth, Virginia, and Chemdal 2000 series available from Chemdal, Inc., Palantine, Illinois. Alternatively, the absorbent core may be made of dual construction, in which case, the SAP may be placed between each layer of the absorbent material. The amount of SAP in the SAP-wood pulp fiber mixture may be from about 5 to about 60 weight percent based on the total weight of the mixture.

The backsheet is preferably made of spunbond nonwoven polypropylene, and is generally coextensive with the coversheet. In some instances, it is desired to provide a film backing, usually a polyethylene film which is liquid, air and preferably vapor impermeable. This film backing is placed under the absorbent core in order to prevent the fluid and body exudates from leaking and otherwise soiling the user's clothing or bed. Polyethylenes suitable as backing film are available from Clopay Plastics, Cincinnati, Ohio.

It may be further desired to include an acquisition layer in order to control absorption and distribution of the fluid and body exudates. The acquisition layer is usually made of chemically bonded nonwoven polypropylene available from American Nonwovens, Columbus, Missouri. Preferably, this layer is substantially coextensive with the same as the width of the absorbent core.

It must be understood that the aforementioned descriptions of the various layers which form the insert 31 do not, in themselves define the novel features of the diapers of this invention. Therefore, said descriptions are not to be interpreted as limitations on the nature or type of the layers used, but are merely referred to for the purpose of general construction of the diapers.

Referring back to the drawings, the insert 31 is shown therein comprises a body side surface 37 which faces, or is in contact with the skin and an opposed surface 39. At its distal end 33, the insert 31 is provided with a pair of opposed and spaced apart hooks 41 and 43 dimensioned to securely engage onto the loops 27, 29 as hereinafter explained.

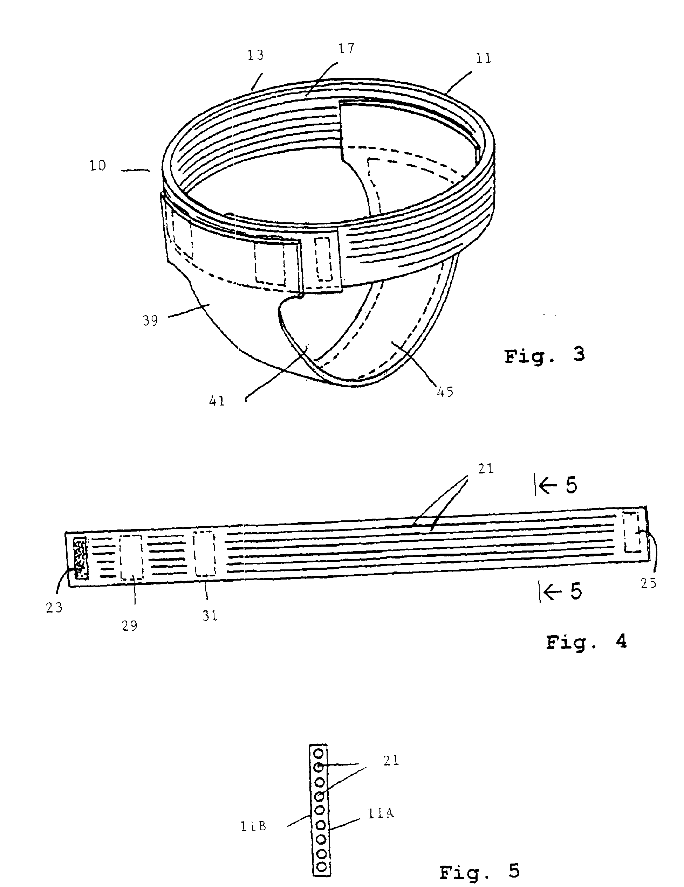

In order to wear the diaper, the belt 11 is wrapped around the torso of the user so that the hook 23 engages the loop 25. Since the belt is stretchable to various degrees, it may be made in a relatively small size in order to realize substantial savings in materials used to define the landing zones. By stretching the belt 11 to a predetermined degree, it can be made to fit a given size torso and thus fit comfortably and tightly by engaging the hook 23 onto the loop 25. Thereafter, the hooks 41 and 43 are securely engaged onto the complementary loops 27 and 29, respectively, when the belt 11 is wrapped around the torso and thus the loops 27 and 29, which are normally located on the reverse surface 19 of the belt 11 will be facing the hooks 41 and 43 in order to engage therein. As it can be further seen from Figure 3, when the diaper 10 is assembled as aforesaid, it will define the leg regions 45 and 47 for inserting the legs of the wearer therethrough.

From the foregoing description of the diaper of this invention, it can be appreciated that relative locations of the hooks and loops on the belt 11 and insert 31 are critical to the proper assembly and advantageous functioning of the diaper. These, together with the degree of elasticity of the belt and the location of the elastic members are the principal features which contribute to the efficacy of the invention and improved performance of the diaper.

In a further preferred construction, the belt 11 is only partly elasticated as shown in Figure 4. In this construction, the areas under the loops preferably are not elasticated.

While the present invention has been described and illustrated by reference to an integral disposable article, i.e., the belt and the insert being fabricated integrally as one piece, such integral construction is not strictly necessary. If desired, the belt and the insert may be fabricated as separate pieces which nevertheless can be used in the manner hereinbefore described. Thus, a suitable adhesive or glue may be applied to the surface of the insert's proximal end and then adhesively securing the insert to the belt before wearing the absorbent article. Other changes and modifications may be made in the construction of such articles which are suggested by the foregoing description and which are nevertheless within the scope of this invention.

## Claims

1. A disposable absorbent article adapted to fit individuals of various torso sizes, said article, in use, comprising a belt member and an absorbent insert member attached to said belt member;
said belt member being at least partly stretchable and having a back waist portion and a front waist portion, each portion having a body side surface and an opposed surface on the reverse side of said body side surface;
an end hook member disposed on the body side surface at one end of said belt member when said belt member is in a generally stretched horizontal position; an end loop member disposed on the body side surface of said belt member at the other end of said belt member, said end hook member and said end loop member being adapted to engage into each other when said belt member is wrapped around the torso of the user, and a pair of spaced apart, generally parallel loop members on said opposite surface of said belt member;
said insert member having a proximal end attached to said belt member and a distal end adapted to be folded onto attachment with said proximal end when the article is worn by the user, said insert member having a body side surface and a surface opposite said body side surface,
a pair of spaced apart, generally parallel hook members disposed at the distal end of said insert member, said hook members being spaced apart to be complementary to and engage in said pair of loops on the opposite surface of said belt member when said belt member is wrapped around the torso of the wearer.

2. An absorbent article as in claim 1 wherein said belt member is stretchable to from about 50 to about 500 percent of its initial length.

3. An absorbent article as in claim 1 wherein said belt member is stretchable to from about 100 to about 300 percent of its initial length.

4. An absorbent article as in any of the claims 1-3 wherein said belt member includes a plurality of spaced apart generally parallel elastic elements.

5. An absorbent article as in any of the claims 1-4 wherein said belt member is non-elasticated under the loops on the surface of said belt member.

6. A disposable absorbent article as in any of the claims 1-5 wherein said belt member is formed of at least two coextensive layers of nonwoven with the elastic elements disposed between said layers.

7. A disposable absorbent article comprising two separate pieces, a first piece defining a belt member adapted to fit individuals of various torso sizes, and a second piece defining an absorbent insert member adapted to be attached to said belt member during use,
said belt member being at least partly stretchable and having a back waist portion and a front waist portion, each portion having a body side surface and an opposed surface on the reverse side of said body side surface;
an end hook member disposed on the body side surface at one end of said belt member when said belt member is in a generally stretched horizontal position; and end loop member disposed on the body side surface of said belt member at the other end of said belt member, said end hook member and said end loop member being adapted to engage into each other when said belt member is wrapped around the torso of the user, and a pair of spaced apart, generally parallel loop members on said opposite surface of said belt member;
said insert member having a proximal end attached to said belt member and a distal end adapted to be folded onto attachment with said proximal end when the article is worn by the user, said insert member having a body side surface and a surface opposite said body side surface,
a pair of spaced apart, generally parallel hook members disposed at the distal end of said insert member, said hook members being spaced apart to be complementary to and engage in said pair of loops on the opposite surface of said belt member when said belt member is wrapped around the torso of the wearer.
